# EUROPÄISCHE PATENTSCHRIFT

(11) **EP 3 233 198 B1**
(45) Veröffentlichungstag und Bekanntmachung des Hinweises auf die Patenterteilung: **18.09.2019**
(21) Anmeldenummer: 15808130.7
(22) Anmeldetag: 03.12.2015
(51) Int. Cl.: A61Q 5/02, A61Q 5/12, A61K 8/19, A61K 8/26

(54) **MITTEL ZUR BEHANDLUNG GEFÄRBTER HAARE**
AGENT FOR TREATING DYED HAIR
AGENT DE TRAITEMENT DE CHEVEUX COLORÉS

(30) Priorität: 17.12.2014 DE 102014226176
(43) Veröffentlichungstag der Anmeldung: 25.10.2017
(73) Patentinhaber: Henkel AG & Co. KGaA, 40589 Düsseldorf (DE)
(72) Erfinder: SCHULZE ZUR WIESCHE, Erik, 22453 Hamburg (DE); KROHN, René, 22851 Norderstedt (DE)
(86) Internationale Anmeldenummer: PCT/EP2015/078452
(87) Internationale Veröffentlichungsnummer: WO 2016/096448

(56) Entgegenhaltungen:
- EP-A2- 1 875 890
- WO-A2-2014/102256
- KR-B1- 101 108 253
- US-A- 2 313 027
- US-A- 3 973 574
- DWECK A C: "Natural ingredients for colouring and styling", INTERNATIONAL JOURNAL OF COSMETIC SCIENCE, KLUWER ACADEMIC PUBLISHERS, DORDRECHT, NL, Bd. 24, 1. Januar 2002 (2002-01-01), Seiten 287-302, XP002319303, ISSN: 0142-5463, DOI: 10.1046/J.1467-2494.2002.00148.X

## Beschreibung

Die Erfindung liegt auf dem Gebiet der Kosmetik und betrifft Haarbehandlungsmittel zum Farbschutz gefärbter Haare, die mindestens ein Alaun und mindestens ein spezifisches weiteres Metallsalz umfassen. Die Erfindung betrifft weiterhin ein Verfahren zum Farbschutz gefärbter Haare.

Produkte zur Veränderung der natürlichen Haarfarbe spielen in der Haarkosmetik eine herausragende Rolle. Man unterscheidet permanente, semipermanente oder temporäre Färbesysteme, die auf chemischen und/oder natürlichen Farbstoffen basieren. Die durch permanente, semipermanente oder temporäre Färbesysteme künstlich erzeugten Haarfarben weisen jedoch den Nachteil auf, dass sie sich - beispielsweise während oder nach der Haarreinigung - in unerwünschter Weise verändern können.

Unter "unerwünschter Veränderung" wird das Verblassen oder Ausbluten sowie der Verlust der Farbbrillanz des durch die jeweilige Färbung erzielten Farbtons der Haare verstanden. Umwelteinflüsse und/oder Sonneneinwirkungen können diese Veränderungen noch verstärken. Es besteht demnach der Bedarf nach kosmetischen Mitteln, mit denen künstlich erzeugte Haarfarben besser stabilisiert werden können.

Haarbehandlungsmittel zum Schutz künstlich erzeugter Haarfarben sind im Prinzip bekannt. Diese Haarbehandlungsmittel basieren üblicherweise auf Silikonen, Polymeren oder bestimmten UV-Filtersubstanzen. In EP 1676604A1 wird ein mehrstufiges Verfahren zur Verbesserung des Farbtons von Haaren beschrieben, bei dem die Haare in einem Schritt mit einem Shampoo gewaschen werden, das u.a. ein wasserlösliches Salz - bevorzugt Natriumsulfat - enthält.

EP 1875890A1 betrifft den Farbschutz und die Pflege gefärbter Haare unter Verwendung von Mitteln, die Lichtfilter, nichtionische Filmbildner und Antioxidantien enthalten.

Die bekannten Zusammensetzungen können jedoch nicht alle Anforderungen an Farbschutzmittel in ausreichendem Maße abdecken. Insbesondere die Färbung sensibler, sehr feiner und/oder geschädigter Haare ist problematisch und sollte zur Verhinderung weiterer Strapazen für die Haare nicht allzu häufig wiederholt werden. In diesen Fällen ist es besonders erstrebenswert, die erzeugte Haarfarbe durch die Behandlung mit geeigneten Mitteln über einen langen Zeitraum - möglichst unverändert - zu erhalten. Darüber hinaus sollten die Haarbehandlungsmittel optimalerweise auch einen Konditionierungsvorteil auf - insbesondere sensiblen, sehr feinen und/oder geschädigten - Haaren bewirken.

Der vorliegenden Erfindung lag die Aufgabe zugrunde, Behandlungsmittel für Keratinfasern, insbesondere für Haare, zur Verfügung zu stellen, mit dem die Haftung von Farbstoffen auf den Fasern gestärkt, und damit die Echtheit der Farbe lange erhalten werden kann.

Idealerweise sollten milde, gut hautverträgliche kosmetische Zusammensetzungen zur Verfügung gestellt werden, die den Haaren neben dem Farbschutz verbesserte optische und haptische Eigenschaften verleihen und die Haare oder die Kopfhaut nicht strapazieren.

Es wurde nun überraschend gefunden, dass die zuvor genannten Aufgaben in hervorragendem Maße durch die Anwendung von Haarbehandlungsmitteln gelöst werden, die mindestens ein Alaun und mindestens ein weiteres spezifisches Metallsalz enthalten.

Die Anwendung dieser Mittel auf farbveränderten Keratinfasern - insbesondere auf gefärbten und/oder gebleichten Haaren - führte zu einer lang anhaltenden und deutlichen Stabilisierung der veränderten Farbe. Der Farbschutzeffekt war besonders nachhaltig auf Keratinfasern, deren Farbe mit oxidativen Mitteln verändert wurde. Insbesondere die Farbtreue auf der sogenannten b-Achse im lab-Farbraum (EN ISO 11664-4) wurde durch die Anwendung der erfindungsgemäßen Mittel verstärkt (geringere Verschiebung in Richtung gelb bzw. blau).

Ein erster Gegenstand der Erfindung ist daher ein Haarbehandlungsmittel zum Farbschutz gefärbter Haare, welches - bezogen auf sein Gewicht -
a) 0,01 bis 4,00 Gew.-% mindestens eines Alauns und
b) 0,01 bis 5,00 Gew.-% mindestens eines organischen oder anorganischen Kupfer-, Zink-, Eisen(II)-, Calcium- und/oder Magnesiumsalzes enthält,
- wobei das Haarbehandlungsmittel einen pH-Wert Im Bereich von 4,0 bis 5,0 aufweist.

Geeignete Haarbehandlungsmittel sind beispielsweise Haarreinigungsmittel wie Shampoos, Haarpflegemittel wie Haarkuren, Spülungen oder Haarpflegesprays sowie Haarstylingmittel wie Haargele, Haarsprays oder Haarwachse. Haarreinigungsmittel und/oder Haarpflegemittel sind besonders bevorzugt.

Erfindungsgemäß bevorzugte Haarbehandlungsmittel enthalten die Wirkstoffe a) und b) bevorzugt in einem kosmetisch akzeptablen Träger. Darunter wird im Rahmen der Erfindung bevorzugt ein wässriger oder wässrig-alkoholischer Träger verstanden.

Der kosmetische Träger enthält bevorzugt mindestens 50 Gew.-%, mehr bevorzugt mindestens 60 Gew.-%, besonders bevorzugt mindestens 70 Gew.-% und insbesondere bevorzugt mindestens 75 Gew.-% Wasser.

Weiterhin kann der kosmetische Träger 0,01 bis 40 Gew.-%, bevorzugt 0,05 bis 30 Gew.-% und insbesondere 0,1 bis 20 Gew.-% mindestens eines Alkohols enthalten.

Geeignete Alkohole sind beispielsweise Ethanol, Ethyldiglykol, 1-Propanol, 2-Propanol, Isopropanol, 1,2-Propylenglycol, 1,3-Propylenglycol, Glycerin, Diglycerin, Triglycerin, 1-Butanol, 2-Butanol, 1,2-Butandiol, 1,3-Butandiol, 1,4-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Polyethylenglycolen, Sorbitol, Sorbitan, Benzylalkohol, Phenoxyethanol oder Mischungen dieser Alkohole.

Besonders bevorzugt sind die wasserlöslichen Alkohole. Insbesondere bevorzugt sind Ethanol, 1,2-Propylenglycol, Glycerin, Benzylalkohol, Phenoxyethanol sowie Mischungen dieser Alkohole.

Es wurde gefunden, dass der Farbschutzeffekt der erfindungsgemäßen Haarbehandlungsmittel durch weitere Faktoren intensiviert werden kann. Zu diesen Faktoren zählen bevorzugt das Gewichtsverhältnis der Komponenten a) und b) in den erfindungsgemäßen Mitteln und die sorgfältige Auswahl besonders bevorzugter Alaune und Salze b), sowie deren Kombination.

In einer ersten bevorzugten Ausführungsform sind erfindungsgemäße Haarbehandlungsmittel bevorzugt, in denen das Gewichtsverhältnis des mindestens einen Alauns a) zu dem mindestens einen Salz b) 4 : 1 bis 1 : 3, bevorzugt 3,5 : 1 bis 1 : 2,5, besonders bevorzugt 3 : 1 bis 1 : 2 und insbesondere 2,5 : 1 bis 1 : 1,5 beträgt.

In einer zweiten bevorzugten Ausführungsform sind erfindungsgemäße Haarbehandlungsmittel bevorzugt, die einen pH-Wert im Bereich von 4,0 bis 5,0, mehr bevorzugt von 4,1 bis 4,9, besonders bevorzugt von 4,2 bis 4,8 und insbesondere von 4,3 bis 4,7 aufweisen. Erfindungsgemäße Mittel, die in diesem pH-Bereich formuliert werden, sind besonders mild, gut (kopf)hautverträglich und verleihen den Keratinfasern, insbesondere Haaren, einen besonderen Glanz und mehr Volumen.

Unter "Alaunen" sind bevorzugt Metallsulfatsalze - und/oder -doppelsalze der allgemeinen Formel M^{I}M^{III}(SO₄)₂ x 12H₂O zu verstehen, in der
- M^{I} bevorzugt für ein Alkalimetallion, ein Ammoniumion oder ein Guanidiniumion,
- M^{III} bevorzugt für ein Aluminium-, Gallium-, Indium-, Titan-, Vanadium-, Chrom-, Mangan-, Eisen-, Cobalt- oder Rhodiumion steht,
und die bevorzugt mit 12 Molekülen Kristallwasser kristallisieren.

Für die erfindungsgemäßen Haarbehandlungsmittel besonders bevorzugte Alaune entsprechen der Formel M^{I}Al(SO₄)₂ x 12 H₂O, in der M^{I} bevorzugt für ein Alkalimetallion, insbesondere für ein Natriumion oder ein Kaliumion, ein Ammoniumion oder ein Guanidiniumion stehen kann. Insbesondere bevorzugt für den Farbschutzeffekt der erfindungsgemäßen Mittel sind NaAl(SO₄)₂ x 12 H₂O, KAl(SO₄)₂ x 12 H₂O, NH₄Al(SO₄)₂ x 12 H₂O sowie Mischungen dieser Alaune.

In einer besonders bevorzugten Ausführungsform enthalten die erfindungsgemäßen Haarbehandlungsmittel daher ein Alaun a) der Formel M^{I}Al(SO₄)₂ x 12 H₂O, in der M' für ein Kalium-, Natrium-, Ammonium- oder Guanidiniumion steht.

In einer weiteren besonders bevorzugten Ausführungsform enthalten die erfindungsgemäßen Haarbehandlungsmittel bezogen auf ihr Gewicht - 0,05 bis 3,50 Gew.-%, bevorzugt 0,10 bis 3,00 Gew.-%, mehr bevorzugt 0,15 bis 2,50 Gew.-%, besonders bevorzugt 0,20 bis 2,25 Gew.-% und insbesondere bevorzugt 0,25 bis 2,00 Gew.-% mindestens eines Alauns a), vorzugsweise eines Kalium-, Natrium- oder Ammoniumalauns der zuvor genannten Formel.

Geeignete Salze b) sind ausgewählt aus Kupfer-, Zink-, Eisen(II)-, Calcium- und/oder Magnesiumsalzen.

Ganz besonders bevorzugt sind Erdalkalimetallkationen und insbesondere bevorzugt Calcium- und Magnesiumsalze.

Besonders geeignete organische Anionen innerhalb dieser Salze können bevorzugt ausgewählt sein aus Acetat-, Lactat-, Succinat-, Citrat-, Tartrat-, Malat-, Maleat-, Oxalat- und/oder Glycolationen. Ganz besonders bevorzugt sind Acetat-, Lactat- und/oder Citratsalze mit den zuvor genannten Kationen. Ganz besonders bevorzugte organische Salze b) sind Calciumlactat, Calciumcitrat, Calciumacetat, Magnesiumlactat, Magnesiumcitrat und/oder Magnesiumacetat. Insbesondere bevorzugt sind Calciumlactat und/oder Magnesiumlactat.

Besonders geeignete anorganische Anionen innerhalb dieser Salze können ausgewählt sein aus Halogenid-, Sulfat-, Phosphat- und/oder Carbonationen. Ganz besonders bevorzugt sind Sulfat- und/oder Halogenidionen wie Chlorid- und Bromidionen.

Insbesondere bevorzugte anorganische Salze b) sind Calciumchlorid, Calciumsulfat, Magnesiumchlorid und/oder Magnesiumsulfat.

Der Gewichtsanteil des mindestens einen Salzes b) am Gesamtgewicht der erfindungsgemäßen Haarbehandlungsmittel beträgt bevorzugt 0,02 bis 4,50 Gew.-%, mehr bevorzugt von 0,05 bis 4,00 Gew.-%, besonders bevorzugt 0,10 bis 3,50 Gew.-% und insbesondere 0,20 bis 3,00 Gew.-%.

In einer weiteren besonders bevorzugten Ausführungsform enthalten die erfindungsgemäßen Haarbehandlungsmittel als Salz b) Calcium- und/oder Magnesiumlactat, insbesondere Calciumlactat.

Es ist erfindungsgemäß auch möglich, dass sich die zuvor genannten Salze b) in den erfindungsgemäßen Haarbehandlungsmitteln erst durch Zugabe einer Säure und eines Salzes bilden. Beispielsweise kann Calciumlactat den erfindungsgemäßen Mitteln als Salz hinzugefügt werden. Es ist aber auch möglich, den Mitteln Milchsäure und beispielsweise Calciumhydroxid oder ein Calciumhalogenid hinzuzufügen.

Für einige Ausführungsformen hat es sich als besonders bevorzugt erwiesen, erst Milchsäure im Überschuss einzusetzen, und anschließend den pH-Wert auf den bevorzugten, zuvor genannten Bereich mit Calciumhydroxid einzustellen. Durch diese Verfahrensweise konnte die Stabilität der Mittel verbessert werden.

Besonders geeignete erfindungsgemäße Haarbehandlungsmittel zur Behandlung keratinischer Fasern sind Haarshampoos, Haarspülungen und/oder Haarkuren, die neben den zuvor genannten Inhaltsstoffen weitere, in den jeweiligen Mittel übliche, Inhaltsstoffe enthalten können. Zu den bevorzugten üblichen Inhaltsstoffen in den zuvor genannten Mitteln zählen Tenside. Weitere übliche Inhaltsstoffe und Beispiele für Standard-Formulierungen der zuvor genannten Haarbehandlungsmittel finden sich beispielsweise in der Monographie Karlheinz Schrader: "Grundlagen und Rezepturen der Kosmetika", 2. Auflage (1989), Hüthig Buch Verlag GmbH Heidelberg, Seiten 676-848.

In einer weiteren bevorzugten Ausführungsform sind erfindungsgemäße Haarbehandlungsmittel dadurch gekennzeichnet, dass sie - bezogen auf ihr Gewicht - 0,05 bis 20 Gew.-% mindestens eines Tensids enthalten.

Haarfarben können sich in der Regel am meisten während oder nach der Haarreinigung verändern. Der Kontakt der Haare mit Wasser und Tensiden, aber auch das Einmassieren eines Shampoos, das Trockenrubbeln nach dem Ausspülen des Shampoos oder die Fönhitze beim anschließenden Trocknungsvorgang können die Haftung der Haarfarbe beeinträchtigen und zu einer unerwünschten Farbveränderung und/oder zu weniger Brillanz der künstlich erzeugten Haarfarbe führen. Es ist daher besonders erstrebenswert, dass der Reinigungsvorgang gefärbter Haare besonders schonend erfolgen kann.

Es wurde gefunden, dass der Reinigungsvorgang gefärbter Haare besonders schonend gelingt, wenn das erfindungsgemäße Haarbehandlungsmittel als Haarshampoo oder als Pflegespülung konfektioniert wird, die direkt am Anschluss an die Haarreinigung angewendet wird.

In einer weiteren bevorzugten Ausführungsform wird das erfindungsgemäße Mittel daher als Haarshampoo konfektioniert. Innerhalb dieser Ausführungsform ist es besonders bevorzugt, wenn Das erfindungsgemäße Mittel - bezogen auf sein Gewicht -1,00 bis 20,00 Gew.-% mindestens eines anionischen Tensids enthält.

Zu der Gruppe der besonders bevorzugten anionischen Tenside zählen beispielsweise:
- Ethercarbonsäuren der Formel R-O-(CH₂-CH₂O)ₓ-CH₂-COOH, in der R eine lineare oder verzweigte, gesättigte oder ungesättigte Alkylgruppe mit 8 bis 30 C-Atomen und x = 0 oder 1 bis 16 ist,
- Acylglutamat- und/oder (Acyl)isethionate mit 8 bis 24 C-Atomen in der Acylgruppe,
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen, und/oder
- Alkylsulfat- und/oder Alkylpolyglykolethersulfatsalze der Formel R-(OCH₂-CH₂)ₓ-OSO₃⁻X⁺, in der R bevorzugt eine lineare oder verzweigte, gesättigte oder ungesättigte Alkylgruppe mit 8 bis 30 C-Atomen, x die Zahl 0 oder 1 bis 12 und X ein Alkali-, Erdalkali-, Ammonium- oder Alkanolaminion bedeutet.

Besonders bevorzugte anionische Tenside sind geradkettige oder verzweigte Alkylethersulfate der zuvor genannten Formel, die einen Alkylrest mit 8 bis 18, insbesondere mit 10 bis 16 C-Atomen, sowie 1 bis 6 und insbesondere 2 bis 4 Ethylenoxideinheiten enthalten. Insbesondere bevorzugt sind die Natrium-, Magnesium und/oder Triethanolaminsalze linearer oder verzweigter Lauryl-, Tridecyl- und/oder Myristylsulfate, die einen Ethoxylierungsgrad von 2 bis 4 aufweisen.

Der Gewichtsanteil der anionischen Tenside am Gesamtgewicht der erfindungsgemäßen Mitteln, die als Haarshampoo konfektioniert werden, beträgt 1,00 bis 20,00 Gew.-%. Mehr bevorzugt ist ein Gewichtsanteil von 2,00 bis 18,00 Gew.-%, besonders bevorzugt von 3,00 bis 17,50 Gew.-% und insbesondere 4,00 bis 15,00 Gew.-%.

In einer weiteren bevorzugten Ausführungsform wird das erfindungsgemäße Mittel als Haarspülung oder als Haarkur konfektioniert. Innerhalb dieser Ausführungsform ist es besonders bevorzugt, wenn das erfindungsgemäße Mittel - bezogen auf sein Gewicht - 0,10 bis 10,00 Gew.-% mindestens eines kationischen Tensids enthält.

Geeignete kationische Tenside sind beispielsweise quartäre Ammoniumverbindungen, Esterquats und/oder Amidoamine.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27, Quaternium-83 und Quaternium-87 bekannten Imidazolium-Verbindungen. Die Alkylketten der oben genannten Tenside weisen bevorzugt 10 bis 18 Kohlenstoffatome auf.

Bei Esterquats handelt es sich um Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart®, Armocare® und Quartamin® vertrieben.

Die Alkylamidoamine werden üblicherweise durch Amidierung natürlicher oder synthetischer Fettsäuren und Fettsäureschnitte mit Dialkylaminoaminen hergestellt. Eine besonders geeignete Verbindung aus dieser Substanzgruppe stellt das unter der Bezeichnung Tegoamid® S 18 im Handel erhältliche Stearamidopropyldimethylamin dar.

Der Gewichtsanteil des mindestens einen kationischen Tensids am Gesamtgewicht der erfindungsgemäßen Mitteln, die als Haarspülung oder als Haarkur konfektioniert werden, beträgt 0,10 bis 10,00 Gew.-%. Mehr bevorzugt ist ein Gewichtsanteil von 0,15 bis 8,00 Gew.-%, besonders bevorzugt von 0,20 bis 7,50 Gew.-% und insbesondere 0,25 bis 5,00 Gew.-%.

Es wurde gefunden, dass die Pflegewirkung der erfindungsgemäßen Mittel noch weiter gesteigert werden kann, wenn Ihnen mindestens ein spezieller haarkonditionierender Wirkstoff, bevorzugt mindestens ein Silikon und/oder mindestens ein kationisches Pflegepolymer, hinzugefügt wird.

In einer weiteren bevorzugten Ausführungsform sind erfindungsgemäße Mittel dadurch gekennzeichnet, dass sie - bezogen auf ihr Gewicht - 0,01 bi 3,00 Gew.-% mindestens eines
Silikons
enthalten.

Noch eine weitere bevorzugte Ausführungsform der erfindungsgemäßen Haarbehandlungsmittel ist dadurch gekennzeichnet, dass sie - bezogen auf ihr Gewicht - 0,01 bis 3 Gew.-% mindestens eines kationischen Polymeren enthalten.

Geeignete Silikone können ausgewählt sein unter:
a) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
b) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
c) linearen Polysiloxan(A)- Polyoxyalkylen(B)- Blockcopoylmeren vom Typ (A-B)ₙ mit n > 3;
d) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
e) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
f) oder deren Gemischen.

Geeignete kationische Polymere sind beispielsweise:
- quaternisierte Cellulose-Derivate, wie sie unter den Bezeichnungen Celquat® und Polymer JR® im Handel erhältlich sind,
- hydrophob modifizierte Cellulosederivate, beispielsweise die unter dem Handelsnamen SoftCat® vertriebenen kationischen Polymere,
- kationische Alkylpolyglycoside,
- kationiserter Honig, beispielsweise das Handelsprodukt Honeyquat® 50,
- kationische Guar-Derivate, wie insbesondere die unter den Handelsnamen Cosmedia®Guar N-Hance® und Jaguar® vertriebenen Produkte,
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und - methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden,
- quaternierter Polyvinylalkohol,
   sowie die unter den Bezeichnungen
- Polyquaternium 2, Polyquaternium 17, Polyquaternium 18, Polyquaternium-24, Polyquaternium 27, Polyquaternium-32, Polyquaternium-37, Polyquaternium 74 und Polyquaternium 89 bekannten Polymere.

Bevorzugte kationische Polymere sind kationische Polysaccharidpolymere wie quaternisierte Cellulosepolymere, hydrophob modifizierte kationische Cellulosederivate und/oder kationische Guarderivate die besonders bevorzugt ausgewählt sind aus den unter den INCI-Bezeichnungen bekannten Polymeren Guar Hydroxypropyltrimonium Chloride, Polyquaternium-10, Polyquaternium-37, Polyquaternium-67 und/oder Polyquaternium-72.

Es wurde gefunden, dass die Farbbrillanz gefärbter Haaren durch die Behandlung mit den erfindungsgemäßen Haarbehandlungsmitteln besonders gut erhalten werden kann, wenn die Mittel als haarkonditionierende Wirkstoffe mindestens ein kationisches Polymer, bevorzugt ein kationisches Polysaccharid, und mindestens ein Silikon enthalten. Durch die Zugabe dieser speziellen Pflegestoffe konnten außerdem die haptischen Eigenschaften wie der Griff und die Geschmeidigkeit der gefärbten Haare verbessert werden.

In einer weiteren bevorzugten Ausführungsform enthalten die erfindungsgemäßen Haarbehandlungsmittel daher zusätzlich als haarkonditionierende Wirkstoffe ein kationisches Polymer - insbesondere ein kationisches Polysaccharid - und ein Silikon.

Die erfindungsgemäßen Mittel sind prinzipiell auf Haaren anwendbar, die mit permanenten, semipermanenten oder temporären Färbesystemen gefärbt wurden. Temporäre Färbesysteme sind jedoch dafür bestimmt, mit der Zeit ausgewaschen zu werden und/oder zu verblassen, deshalb sind die erfindungsgemäßen Mittel besonders geeignet für die Anwendung auf Haaren, die mit permanenten oder oxidativen Haarfärbemitteln gefärbt wurden.

In einer weiteren bevorzugten Ausführungsform sind daher erfindungsgemäße Mittel besonders bevorzugt, die vor oder nach einer oxidativen Behandlung (Färbung) von Haaren angewendet werden.

Die erfindungsgemäßen Mittel können neben den zuvor genannten Inhaltsstoffen weitere Wirkstoffe enthalten, die ihnen zusätzliche vorteilhafte Eigenschaften verleihen bzw. diese unterstützen. Beispiele für solche weiteren Wirkstoffe sind natürliche und/oder mineralische Öl-, Fett- oder Wachskomponenten (die keine Silikone sind), Vitamine und/oder Proteinhydrolysate.

Unter geeigneten Proteinhydrolysaten sind Produktgemische zu verstehen, die durch sauer, basisch oder enzymatisch katalysierten Abbau von Proteinen (Eiweißen) erhalten werden können.

Es können Proteinhydrolysate pflanzlichen, tierischen und/oder marinen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.

Bevorzugt sind Proteinhydrolysate pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex) und Crotein® (Croda) erhältlich.

Einsetzbar sind auch kationisierte Proteinhydrolysate, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten, stammen kann. Die den kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die kationischen Proteinhydrolysate und -derivate seien die unter den INCI - Bezeichnungen bekannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein.

Das oder die Proteinhydrolysate kann (können) in den erfindungsgmäßen Mitteln - bezogen auf deren Gesamtgewicht - bevorzugt in Mengen von 0,01 bis 5 Gew.-%, mehr bevorzugt von 0,025 bis 3 Gew.-% und insbesondere von 0,05 bis 2 Gew.-% eingesetzt werden.

Unter geeigneten Vitaminen sind bevorzugt die folgenden Vitamine, Provitamine und Vitaminvorstufen sowie deren Derivate zu verstehen:
- Vitamin A: zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht.
- Vitamin B: zur Vitamin B-Gruppe oder zu dem Vitamin B-Komplex gehören u. a.
   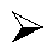 Vitamin B₁ (Thiamin)
   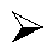 Vitamin B₂ (Riboflavin)
   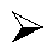 Vitamin B₃. Unter dieser Bezeichnung werden häufig die Verbindungen Nicotinsäure und Nicotinsäureamid (Niacinamid) geführt.
   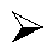 Vitamin B₅ (Pantothensäure und Panthenol). Im Rahmen dieser Gruppe wird bevorzugt das Panthenol eingesetzt. Einsetzbare Derivate des Panthenols sind insbesondere die Ester und Ether des Panthenols, Pantolacton sowie kationisch derivatisierte Panthenole. Einzelne Vertreter sind beispielsweise das Panthenoltriacetat, der Panthenolmonoethylether und dessen Monoacetat sowie kationische Panthenolderivate.
   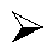 Vitamin B₆ (Pyridoxin sowie Pyridoxamin und Pyridoxal).
- Vitamin C (Ascorbinsäure): die Verwendung in Form des Palmitinsäureesters, der Glucoside oder Phosphate kann bevorzugt sein. Die Verwendung in Kombination mit Tocopherolen kann ebenfalls bevorzugt sein.
- Vitamin E (Tocopherole, insbesondere α-Tocopherol).
- Vitamin F: unter dem Begriff "Vitamin F" werden üblicherweise essentielle Fettsäuren, insbesondere Linolsäure, Linolensäure und Arachidonsäure, verstanden.
- Vitamin H: Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat.

Die erfindungsgemäßen Mittel können bevorzugt Vitamine, Provitamine und Vitaminvorstufen aus den Gruppen A, B, E und H enthalten.

Insbesondere bevorzugt sind Nicotinsäureamid, Biotin, Pantolacton und/oder Panthenol.

Vitamine, Vitaminderivate und/oder Vitaminvorstufen können in den erfindungsgemäßen Mitteln - bezogen auf deren Gesamtgewicht - bevorzugt in einer Menge von 0,001 bis 2 Gew.-%, besonders bevorzugt von 0,005 bis 1 Gew.-% und insbesondere von 0,01 bis 0,5 Gew.-% eingesetzt werden.

Unter geeigneten natürlichen (pflanzlichen) Ölen werden üblicherweise Triglyceride und Mischungen von Triglyceriden verstanden. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaoabutter und/oder Shea-Butter.

Als mineralische Öle kommen insbesondere Mineralöle, Paraffin- und Isoparaffinöle sowie synthetische Kohlenwasserstoffe zum Einsatz. Ein Beispiel für einen einsetzbaren Kohlenwasserstoff ist beispielsweise das als Handelsprodukt erhältliche 1,3-Di-(2-ethylhexyl)-cyclohexan (Cetiol® S).

Als Ölkomponente kann weiterhin ein Dialkylether dienen.

Einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Din-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert.-butylether, Di-iso-pentylether, Di-3-ethyldecylether, tert.-Butyl-n-octylether, iso-Pentyl-n-octylether und 2-Methylpentyl-n-octylether.

Besonders bevorzugt ist der Di-n-octylether, der im Handel unter der Bezeichnung Cetiol® OE erhältlich ist.

Unter Fettstoffen sind zu verstehen Fettsäuren, Fettalkohole sowie natürliche und synthetische Wachse, welche sowohl in fester Form als auch flüssig in wässriger Dispersion vorliegen können.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol®871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.

Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol®, z.B. Stenol® 1618 oder Lanette®, z.B. Lanette® O oder Lorol®, z.B. Lorol® C8, Lorol® C14, Lorol® C18, Lorol® C8-18, HD-Ocenol®, Crodacol®, z.B. Crodacol® CS, Novol®, Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona®, White Swan®, Coronet® oder Fluilan® käuflich zu erwerben sind, eingesetzt werden.

Als natürliche oder synthetische Wachse können eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Weitere Fettstoffe sind beispielsweise
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.
   Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren
   technische Mischungen. Besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen,
- Glycerincarbonat oder Dicaprylylcarbonat (Cetiol® CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls® 90-018, Monomuls® 90-L12, Cetiol® HE oder Cutina® MD.

Der Gewichtsanteil der Öl-, Wachs- und/oder Fettkomponenten am Gesamtgewicht der erfindungsgemäßen Mittel beträgt bevorzugt 0,01 bis 10 Gew.-%, besonders bevorzugt 0,025 bis 7,5 Gew.-% und insbesondere 0,05 bis 5 Gew.-%.

In einer vierten bevorzugten Ausführungsform sind erfindungsgemäße Mittel dadurch gekennzeichnet, dass sie - bezogen auf ihr Gewicht -
a) 0,05 bis 3,50 Gew.-%, bevorzugt 0,10 bis 3,00 Gew.-%, mehr bevorzugt 0,15 bis 2,50 Gew.-%, besonders bevorzugt 0,20 bis 2,25 Gew.-% und insbesondere bevorzugt 0,25 bis 2,00 Gew.-% mindestens eines Alauns der Formel M^{I}M^{III}(SO₄)₂ x 12H₂O enthalten, in der
   - M^{I} bevorzugt für ein Alkalimetallion, ein Ammoniumion oder ein Guanidiniumion, und
   - M^{III} bevorzugt für ein Aluminium-, Gallium-, Indium-, Titan-, Vanadium-, Chrom-, Mangan-, Eisen-, Cobalt- oder Rhodiumion steht,
b) 0,02 bis 4,50 Gew.-%, mehr bevorzugt von 0,05 bis 4,00 Gew.-%, besonders bevorzugt 0,10 bis 3,50 Gew.-% und insbesondere 0,20 bis 3,00 Gew.-% mindestens eines organischen Kupfer-, Zink-, Eisen(II)-, Calcium- und/oder Magnesiumsalzes, insbesondere eines Milchsäuresalzes,
c) 2,00 bis 18,00 Gew.-%, besonders bevorzugt von 3,00 bis 17,50 Gew.-% und insbesondere 4,00 bis 15,00 Gew.-% mindestens eines anionischen Tensids und/oder 0,15 bis 8,00 Gew.-%, besonders bevorzugt von 0,20 bis 7,50 Gew.-% und insbesondere 0,25 bis 5,00 Gew.-% mindestens eines kationischen Tensids, und
d) 0,01 bis 3,00 Gew.-%, mehr bevorzugt 0,05 bis 2,50 Gew.-% und insbesondere 0,10 bis 2,00 Gew.-% mindestens eines kationischen Polymeren und/oder 0,01 bis 3,00 Gew.-%, mehr bevorzugt 0,05 bis 2,50 Gew.-% und insbesondere 0,10 bis 2,00 Gew.-% mindestens eines Silikons enthalten, und
einen pH-Wert im Bereich von 4,0 bis 5,0, mehr bevorzugt von 4,1 bis 4,9, besonders bevorzugt von 4,2 bis 4,8 und insbesondere von 4,3 bis 4,7 aufweisen.

Innerhalb dieser Ausführungsform sind erfindungsgemäße Mittel besonders bevorzugt, die
a) 0,05 bis 3,50 Gew.-%, bevorzugt 0,10 bis 3,00 Gew.-%, mehr bevorzugt 0,15 bis 2,50 Gew.-%, besonders bevorzugt 0,20 bis 2,25 Gew.-% und insbesondere bevorzugt 0,25 bis 2,00 Gew.-% mindestens eines Alauns der Formel M^{I}Al(SO₄)₂ x 12 H₂O, in der M^{I} bevorzugt für ein Alkalimetallion, insbesondere für ein Natriumion oder ein Kaliumion, ein Ammoniumion oder ein Guanidiniumion stehen,
b) 0,02 bis 4,50 Gew.-%, mehr bevorzugt von 0,05 bis 4,00 Gew.-%, besonders bevorzugt 0,10 bis 3,50 Gew.-% und insbesondere 0,20 bis 3,00 Gew.-% Calcium- und/oder Magnesiumlactat,
c) 2,00 bis 18,00 Gew.-%, besonders bevorzugt von 3,00 bis 17,50 Gew.-% und insbesondere 4,00 bis 15,00 Gew.-% mindestens eines Alkylsulfat- und/oder Alkylpolyglykolethersulfatsalzes der Formel R-(OCH₂-CH₂)ₓ-OS0₃⁻X⁺, in der R bevorzugt eine lineare oder verzweigte, gesättigte oder ungesättigte Alkylgruppe mit 8 bis 30 C-Atomen, x die Zahl 0 oder 1 bis 12 und X ein Alkali-, Erdalkali-, Ammonium- oder Alkanolaminion bedeutet, und
d) 0,01 bis 3,00 Gew.-%, mehr bevorzugt 0,05 bis 2,50 Gew.-% und insbesondere 0,10 bis 2,00 Gew.-% mindestens eines der unter den INCI-Bezeichnungen Polyquaternium-10, Guar Hydroxypropyltrimonium Chloride, Polyquaternium-37, Polyquaternium-67 und/oder Polyquaternium-72 bekannten kationischen Polymere und/oder 0,01 bis 3,00 Gew.-%, mehr bevorzugt 0,05 bis 2,50 Gew.-% und insbesondere 0,10 bis 2,00 Gew.-% mindestens eines unter der INCI-Bezeichnung Dimethicone bekannten Silikons enthalten.

Innerhalb dieser Ausführungsform sind weiterhin erfindungsgemäße Mittel besonders bevorzugt, die
a) 0,05 bis 3,50 Gew.-%, bevorzugt 0,10 bis 3,00 Gew.-%, mehr bevorzugt 0,15 bis 2,50 Gew.-%, besonders bevorzugt 0,20 bis 2,25 Gew.-% und insbesondere bevorzugt 0,25 bis 2,00 Gew.-% mindestens eines Alauns der Formel M^{I}Al(SO₄)₂ x 12H₂O, in der M^{I} bevorzugt für ein Alkalimetallion, insbesondere für ein Natriumion oder ein Kaliumion, ein Ammoniumion oder ein Guanidiniumion stehen,
b) 0,02 bis 4,50 Gew.-%, mehr bevorzugt von 0,05 bis 4,00 Gew.-%, besonders bevorzugt 0,10 bis 3,50 Gew.-% und insbesondere 0,20 bis 3,00 Gew.-% Calcium- und/oder Magnesiumlactat,
c) 0,15 bis 8,00 Gew.-%, besonders bevorzugt von 0,20 bis 7,50 Gew.-% und insbesondere 0,25 bis 5,00 Gew.-% Cetyltrimethylammoniumchlorid, Stearyltrimethylammoniumchlorid, Behentrimethylammoniumchlorid, Distearyldimethylammoniumchlorid, Lauryldimethylammoniumchlorid, Lauryldimethylbenzylammoniumchlorid und/oder Tricetylmethylammoniumchlorid, und
e) 0,01 bis 3,00 Gew.-%, mehr bevorzugt 0,05 bis 2,50 Gew.-% und insbesondere 0,10 bis 2,00 Gew.-% mindestens eines der unter den INCI-Bezeichnungen Polyquaternium-10, Guar Hydroxypropyltrimonium Chloride, Polyquaternium-37, Polyquaternium-67 und/oder Polyquaternium-72 bekannten kationischen Polymere und/oder Polyquaternium-72 bekannten kationischen Polymere und/oder 0,01 bis 3,00 Gew.-%, mehr bevorzugt 0,05 bis 2,50 Gew.-% und insbesondere 0,10 bis 2,00 Gew.-% mindestens eines unter der INCI-Bezeichnung Dimethicone bekannten Silikons enthalten.

Als weitere fakultative Inhaltsstoffe können die erfindungsgemäßen Mittel eine oder mehrere Verbindungen aus den folgenden Gruppen enthalten:
- nichtionische und/oder amphotere Tenside,
- Antischuppenwirkstoffe,
- Perlglanzmittel und/oder Trübungsmittel.

Zu der Gruppe der geeigneten nichtionischen Tenside zählen beispielsweise:
- C₈-C₃₀-Fettsäuremono- und -diester von Anlagerungsprodukten von 1 bis 30 Mol Ethylenoxid an Glycerin,
- Aminoxide,
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- Sorbitanfettsäureester und Anlagerungeprodukte von Ethylenoxid an Sorbitanfettsäureester wie beispielsweise Polysorbate,
- Fettsäurealkanolamide,
- Zuckerfettsäureester und Anlagerungsprodukte von Ethylenoxid an Zuckerfettsäureester,
- Anlagerungsprodukte von Ethylenoxid an Fettsäurealkanolamide und Fettamine und/oder
- Alkylpolyglucoside der allgemeinen Formel RO-[G]ₓ, in der R für einen Alkyl- und/oder Alkenylrest mit 4 bis 22 C-Atomen, G für einen Zuckerrest mit 5 oder 6 C-Atomen und x für Zahlen von 1 bis 10 steht. Besonders geeignete Alkylpolyglucoside leiten sich von Aldosen und/oder Ketosen mit 5 oder 6 Kohlenstoffatomen, vorzugsweise von Glucose ab. Der Rest R steht besonders bevorzugt für einen Alkylrest mit 6 bis 20 und insbesondere mit 8 bis 18 Kohlenstoffatomen. Die Indexzahl x steht in der allgemeinen Formel RO-[G][x] für den Oligomerisierungsgrad (DP), d. h. für die Verteilung der Mono- und Oligoglycoside. Die Indexzahl x weist vorzugsweise einen Wert im Bereich von 1 bis 6, besonders bevorzugt im Bereich von 1 bis 3 auf, wobei es sich dabei um keine ganze Zahl, sondern um eine gebrochene Zahl handeln kann, die analytisch ermittelt werden kann. Insbesondere bevorzugte Alkylpolyglucoside weisen einen Oligomerisierungsgrad zwischen 1,2 und 1,5 auf.
Insbesondere geeignete Alkylpolyglucoside sind bekannt und im Handel von verschiedenen Anbietern erhältlich unter den INCI-Bezeichnungen Decyl Glucoside, Lauryl Glucoside und Coco Glucoside.

Besonders geeignete nichtionische Tenside/Emulgatoren sind Alkylpolyglucoside, insbesondere Alkylpolyglucoside auf der Basis von gehärtetem C₁₀₋₁₄-Kokosalkohol mit einem DP von 1-3, wie sie beispielsweise unter der INCI-Bezeichnung "Coco-Glucoside" im Handel erhältlich sind.

Das (oder die) nichtionische(n) Tensid(e) wird (werden) in den erfindungsgemäßen Mitteln - bezogen auf deren Gesamtgewicht - bevorzugt in einer Menge von 0,10 bis 15,00 Gew.-%, mehr bevorzugt von 0,25 bis 12,50 Gew.-%, besonders bevorzugt von 0,40 bis 10,00 Gew.-% und insbesondere von 0,50 bis 5,00 Gew.-% eingesetzt.

Zu der Gruppe der geeigneten amphoteren und/oder zwitterionischen Tenside zählen die sogenannten Betaine wie die N-Alkyl-N,N-dimethylammonium-glycinate, beispielsweise das Kokosalkyldimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat.

Unter amphoteren Tensiden werden solche oberflächenaktiven Verbindungen verstanden, die zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Aminopropionate, Aminoglycinate, Imidazoliniumbetaine und Sulfobetaine.

Besonders bevorzugte amphotere Tenside sind das N-Kokosalkylaminopropionat, das Kokosacylaminoethylaminopropionat, das unter der INCI-Bezeichnung Cocamidopropyl Betaine bekannte Fettsäureamid-Derivat und das C₁₂ - C₁₈ - Acylsarcosin.

Die Gesamtmenge an amphoteren und/oder zwitterionischen Tensiden in den erfindungsgemäßen Mitteln beträgt bevorzugt 1,00 bis 15,00 Gew.%, mehr bevorzugt von 1,25 bis 12,50 Gew.%, besonders bevorzugt 1,50 bis 10,00 Gew.% und insbesondere 1,75 bis 7,50 Gew.%, wobei sich die Mengenangaben auf das Gesamtgewicht der Mittel beziehen.

Antischuppenwirkstoffe können in den erfindungsgemäßen Mitteln - bezogen auf deren Gesamtgewicht - bevorzugt in Mengen von 0,01 bis 10 Gew.-%, mehr bevorzugt von 0,025 bis 7,5 Gew.-%, besonders bevorzugt von 0,05 bis 5 Gew.-% und insbesondere von 0,075 bis 3 Gew.-% eingesetzt werden.

Geeignete Antischuppenwirkstoffe können ausgewählt sein aus Piroctone Olamine, Climbazol, Zink Pyrithion, Ketoconazole, Salicylsäure, Schwefel, Selensulfid, Teerpräparaten, Undecensäurederivaten, Klettenwurzelextrakten, Pappelextrakten, Brennesselextrakten, Walnussschalenextrakten, Birkenextrakten, Weidenrindenextrakten, Rosmarinextrakten und/oder Arnikaextrakten. Bevorzugt sind Climbazol, Zink Pyrithion, Piroctone Olamine und/oder Salicylsäure. Insbesondere bevorzugt ist Zinkpyrithion.

Geeignete Trübungs- und/oder Perlglanzmittel können in den erfindungsgemäßen Mitteln - bezogen auf deren Gesamtgewicht - bevorzugt in Mengen von (jeweils) 0,001 bis 5 Gew.-%, mehr bevorzugt von 0,005 bis 4 Gew.-%, besonders bevorzugt von 0,01 bis 3 Gew.-% und insbesondere von 0,05 bis 2 Gew.-% eingesetzt werden.

Unter geeigneten Perlglanzmitteln und Trübungsmitteln sind beispielsweise
- Mono- und/oder Diester des Ethylenglycols, 1,2-Propandiols und/oder Glycerins mit C₈-C₂₄-Fettsäuren,
- Ester aus Polyethylenglycolen mit C₈-C₂₄-Fettsäuren,
- TiO₂ oder TiO₂-beschichtetes synthetisches oder natürliches Mica und/oder
- Styrol/Acrylat-Copolymere zu verstehen.

Besonders geeignet sind die unter den INCI-Bezeichnungen bekannten Trübungs- und/oder Perlglanzmittel: Glycol Distearate, wie beispielsweise das Handelsprodukt Cutina® AGS der Firma Cognis, Glycol Monostearate, wie beispielsweise das Handelsprodukt Cutina® EGMS der Firma Cognis, PEG-3 Distearate, wie beispielsweise das Handelsprodukt Genapol® TS der Firma Clariant, PEG-2 Distearate, wie beispielsweise das Handelsprodukt Kessco® DEGMS der Firma Akzo Nobel, Propylen Glycol Stearate, wie beispielsweise das Handelsprodukt Tegin® P der Firma Goldschmidt und/oder Styrol/Acrylates-Copolymere wie beispielsweise die Handelsprodukte Joncryl® 67 der Firma Johnson Polymers, Suprawal® WS der Firma BASF und/oder Acusol® OP 301 der Firma Rohm & Haas.

Weitere Wirk-, Hilfs- und Zusatzstoffe, die in den erfindungsgemäßen Mitteln enthalten können, sind beispielsweise:
- Pflanzenextrakte,
- Feuchthaltemittel,
- Parfums,
- UV-Filter,
- Verdickungsmittel wie Gelatine oder Pflanzengumme, beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone und Schichtsilikate wie z. B. Bentonit oder vollsynthetische Hydrokolloide wie z. B. Polyvinylalkohol, die Ca-, Mg- oder Zn- Seifen,
- faserstrukturverbessernde Wirkstoffe, insbesondere Mono-, Di- und Oligosaccharide wie beispielsweise Glucose, Galactose, Fructose, Fruchtzucker und Lactose,
- Farbstoffe zum Anfärben des Mittels,
- Wirkstoffe wie Bisabolol,
- Ceramide,
- Treibmittel wie Propan-Butan-Gemische, N₂O, Dimethylether, CO₂ und Luft,
- Antioxidantien,
- Konservierungsmittel, wie beispielsweise Natriumbenzoat oder Salicylsäure,
- Viskositätsregler wie Salze (NaCl).

Ein zweiter Gegenstand der Erfindung ist ein Verfahren zum Farbschutz gefärbter Haare, bei dem eine kosmetische Zusammensetzung, die einen pH-Wert Im Bereich von 4,0 bis 5,0 aufweist, und die - bezogen auf ihr Gewicht -
a) 0,01 bis 4,00 Gew.-% mindestens eines Alauns und
b) 0,01 bis 5,00 Gew.-% mindestens eines organischen oder anorganischen Kupfer-, Zink-, Eisen(II)-, Calcium- und/oder Magnesiumsalzes enthält,
innerhalb eines Zeitraums von 5 Sekunden bis 24 Stunden vor oder nach einer Färbung auf die Haare aufgebracht wird.

Für bevorzugte Ausführungsformen des erfindungsgemäßen Verfahrens gilt mutatis mutandis das zu den erfindungsgemäßen Mitteln Gesagte Der Schutzumfang der Erfindung ist durch die beigefügten Ansprüche definiert.

### Beispiele:

Es wurden die folgenden erfindungsgemäßen Mittel hergestellt (die Mengenangaben beziehen sich auf Gew.-%):

**a) Farbschutz-Shampoos**

| | **Shampoo 1** | **Shampoo 2** | **Shampoo 3** |
|---|---|---|---|
| Sodium Laureth Sulfate | 11,00 | 11,00 | 10,00 |
| Cocamidopropyl Betaine | 1,00 | 1,50 | 3,00 |
| Disodium Cocoamphodiacetate | 0,50 | | |
| Cocamide MEA | 0,50 | 0,50 | |
| PEG-12 Dimethicone | 0,50 | 0,30 | |
| Glycol Distearate | 1,20 | | |
| PEG-7 Glyceryl Cocoate | 0,40 | 0,60 | 0,80 |
| Polyquaternium-10 | 0,90 | | 0,60 |
| Guar Hydroxypropyltrimonium Chloride | | 0,60 | |
| Panthenol | 0,30 | 0,20 | 0,20 |
| Dimethicone | 0,10 | | 0,50 |
| Hydrogenated Castor Oil | 0,20 | 0,10 | 0,30 |
| Calciumlactate | 0,40 | 0,50 | 0,40 |
| KAI(SO₄)₂ x 12 H₂O | 0,80 | 1,00 | |
| NH₄Al(SO₄)₂ x 12 H₂O | | | 0,75 |
| Konservierungsmittel, Parfum, evtl. Säuerungsmittel | q.s. | q.s. | q.s. |
| Wasser | ad 100 | ad 100 | ad 100 |
| pH | 4,5 | 4,4 | 4,5 |

Oxidativ gefärbte Haare weisen nach der wiederholten Reinigung (10-20 Reinigungsvorgänge) mit den o.g. Shampoos eine brillante Haarfarbe auf, deren Echtheitseigenschaften sich nicht wesentlich verändern. Zudem weisen die gefärbten Haare einen optisch ansprechenden Glanz und einen besonders weichen Griff auf.

**b) Conditioner**

| | **Conditioner 1** | **Conditioner 2** | **Conditioner 3** |
|---|---|---|---|
| Cetearvl Alcohol | 2,50 | 2,50 | 3,00 |
| Quaternium-87 | 2,00 | 2,00 | 2,50 |
| Lactic Acid | 1,60 | | |
| Propylene Glycol | 0,50 | 0,50 | 1,00 |
| Isopropyl Mvristate | 0,30 | 0,30 | 0,50 |
| Distearoylethyl Hydroxyethylmonium Methosulfate | 0,50 | 0,50 | 0,50 |
| Calcium Hydroxide | 0,54 | | |
| Phenoxyethanol | 0,40 | 0,40 | 0,60 |
| Stearamidopropyl Dimethylamine | 0,30 | 0,30 | 0,40 |
| Parfum | 0,25 | 0,25 | 0,25 |
| Sodium Methylparaben | 0,20 | 0,20 | 0,20 |
| Polyquaternium-37 | 0,20 | 0,20 | 0,20 |
| Dicaprylyl Carbonate | 0,20 | 0,20 | 0,20 |
| Panthenol | 0,10 | 0,10 | 0,10 |
| Benzophenone-4 | 0,05 | 0,05 | 0,05 |
| Amodimethicone/Morpholinomethyl Silsesquioxane Copolymer | 0,02 | 0,02 | 0,02 |
| Glvcerin | 0,01 | 0,01 | 0,01 |
| Hydrolyzed Keratin | 0,01 | 0,01 | 0,01 |
| Calciumlactate | 0,80 | 1,40 | 1,40 |
| KAl(SO₄)₂ x 12 H₂O | 0,80 | 1,80 | |
| NH₄Al(SO₄)₂ x 12 H₂O | | | 2,00 |
| Konservierungsmittel, Parfum, evtl. Säuerungsmittel | q.s. | q.s. | q.s. |
| Wasser | ad 100 | ad 100 | ad 100 |
| pH | 4,3 | 4,3 | 4,5 |

## Patentansprüche

1. Haarbehandlungsmittel zum Farbschutz gefärbter Haare, enthaltend - bezogen auf sein Gewicht -
a. 0,01 bis 4,00 Gew.-% mindestens eines Alauns und
b. 0,01 bis 5,00 Gew.-% mindestens eines organischen oder anorganischen Kupfer-, Zink-, Eisen(II)-, Calcium- und/oder Magnesiumsalzes,
- wobei das Haarbehandlungsmittel einen pH-Wert Im Bereich von 4,0 bis 5,0 aufweist.

2. Haarbehandlungsmittel nach Anspruch 1, **dadurch gekennzeichnet dass** das Gewichtsverhältnis des mindestens einen Alauns a) zu dem mindestens einen Salz b) 4: 1 bis 1 : 3, bevorzugt 3,5 : 1 bis 1 : 2,5, besonders bevorzugt 3 : 1 bis 1 : 2 und insbesondere 2,5 : 1 bis 1 : 1,5 beträgt.

3. Haarbehandlungsmittel nach einem der vorhergehenden Ansprüche, **dadurch gekennzeichnet, dass** es einen pH-Wert im Bereich von 4,1 bis 4,9, besonders bevorzugt von 4,2 bis 4,8 und insbesondere bevorzugt von 4,3 bis 4,7 aufweist.

4. Haarbehandlungsmittel nach einem der vorhergehenden Ansprüche, **dadurch gekennzeichnet, dass** das Alaun a) der Formel M^{I}Al(SO₄)₂ entspricht, in der M^{I} für ein Kalium- Natrium- oder Ammoniumion bzw. Guanidiniumion steht.

5. Haarbehandlungsmittel nach einem der vorhergehenden Ansprüche, **dadurch gekennzeichnet, dass** es - bezogen auf sein Gewicht - 0,05 bis 3,50 Gew.-%, bevorzugt 0,10 bis 3,00 Gew.-%, mehr bevorzugt 0,15 bis 2,50 Gew.-%, besonders bevorzugt 0,20 bis 2,25 Gew.-% und insbesondere bevorzugt 0,25 bis 2,00 Gew.-% mindestens eines Alauns, vorzugsweise eines Kalium-, Natrium- oder Ammoniumalauns, enthält.

6. Haarbehandlungsmittel nach einem der vorhergehenden Ansprüche, **dadurch gekennzeichnet, dass** als Salz Calcium- und/oder Magnesiumlactat eingesetzt wird

7. Haarbehandlungsmittel nach einem der vorhergehenden Ansprüche, **dadurch gekennzeichnet, dass** es - bezogen auf sein Gewicht - 0,05 bis 20 Gew.-% mindestens eines Tensids enthält.

8. Haarbehandlungsmittel nach Anspruch 7, **dadurch gekennzeichnet, dass** es - bezogen auf sein Gewicht - 1,00 bis 20 Gew.-% mindestens eines anionischen Tensids enthält.

9. Haarbehandlungsmittel nach Anspruch 7, **dadurch gekennzeichnet, dass** es - bezogen auf sein Gewicht - 0,1 bis 10 Gew.-% mindestens eines kationischen Tensids enthält.

10. Haarbehandlungsmittel nach einem der vorhergehenden Ansprüche, **dadurch gekennzeichnet, dass** es - bezogen auf sein Gewicht - 0,01 bis 3 Gew.-% mindestens eines Silikons enthält.

11. Haarbehandlungsmittel nach einem der vorhergehenden Ansprüche, **dadurch gekennzeichnet, dass** es - bezogen auf sein Gewicht - 0,01 bis 3 Gew.-% mindestens eines kationischen Polymers enthält.

12. Haarbehandlungsmittel nach einem der vorhergehenden Ansprüche, **dadurch gekennzeichnet, dass** es vor oder nach einer oxidativen Behandlung (Färbung) von keratinischen Fasern angewendet wird.

13. Verfahren zum Farbschutz gefärbter keratinischer Fasern, bei dem eine kosmetische Zusammensetzung, die einen pH-Wert Im Bereich von 4,0 bis 5,0 aufweist, und die - bezogen auf ihr Gewicht -
a. 0,01 bis 4,00 Gew.-% mindestens eines Alauns und
b. 0,01 bis 5,00 Gew.-% mindestens eines organischen oder anorganischen Kupfer-, Zink-, Eisen(II)-, Calcium- und/oder Magnesiumsalzes enthält,
innerhalb eines Zeitraums von 5 Sekunden bis 24 Stunden vor oder nach einer Färbung auf die keratinischen Fasern aufgebracht wird.

## Claims

1. A hair treatment agent for the color protection of dyed hair, containing, based on its weight,
a. 0.01 to 4.00 wt.% of at least one alum and
b. 0.01 to 5.00 wt.% of at least one organic or inorganic copper, zinc, iron (II), calcium and/or magnesium salt,
- wherein the hair treatment agent has a pH in the range of from 4.0 to 5.0.

2. The hair treatment agent according to claim 1, **characterized in that** the weight ratio of the at least one alum a) to the at least one salt b) is from 4:1 to 1:3, preferably 3.5:1 to 1:2.5, particularly preferably 3:1 to 1:2 and in particular 2.5:1 to 1:1.5.

3. The hair treatment agent according to one of the preceding claims, **characterized in that** it has a pH in the range of from 4.1 to 4.9, more preferably from 4.2 to 4.8, and particularly preferably from 4.3 to 4.7.

4. The hair treatment agent according to one of the preceding claims, **characterized in that** the alum a) corresponds to the formula M^{I}Al(SO₄)₂, in which M^{I} is a potassium, sodium or ammonium ion or guanidinium ion.

5. The hair treatment agent according to one of the preceding claims, **characterized in that** it contains, based on its weight, 0.05 to 3.50 wt.%, preferably 0.10 to 3.00 wt.%, more preferably 0.15 to 2.50 wt.%, even more preferably 0.20 to 2.25 wt.%, and particularly preferably 0.25 to 2.00 wt.% of at least one alum, preferably a potassium, sodium or ammonium alum.

6. The hair treatment agent according to one of the preceding claims, **characterized in that** calcium lactate and/or magnesium lactate is used as a salt.

7. The hair treatment agent according to one of the preceding claims, **characterized in that** it contains, based on its weight, 0.05 to 20 wt.% of at least one surfactant.

8. The hair treatment agent according to claim 7, **characterized in that** it contains, based on its weight, 1.00 to 20 wt.% of at least one anionic surfactant.

9. The hair treatment agent according to claim 7, **characterized in that** it contains, based on its weight, 0.1 to 10 wt.% of at least one cationic surfactant.

10. The hair treatment agent according to one of the preceding claims, **characterized in that** it contains, based on its weight, 0.01 to 3 wt.% of at least one silicone.

11. The hair treatment agent according to one of the preceding claims, **characterized in that** it contains, based on its weight, 0.01 to 3 wt.% of at least one cationic polymer.

12. The hair treatment agent according to one of the preceding claims, **characterized in that** it is applied before or after an oxidative treatment (dyeing) of keratin fibers.

13. A method for the color protection of dyed keratin fibers, in which a cosmetic composition which has a pH in the range of from 4.0 to 5.0 and contains, based on its weight,
a. 0.01 to 4.00 wt.% of at least one alum and
b. 0.01 to 5.00 wt.% of at least one organic or inorganic copper, zinc, iron (II), calcium and/or magnesium salt,
is applied to the keratin fibers within a period of 5 seconds to 24 hours before or after dyeing.

## Revendications

1. Agent de traitement capillaire pour la protection de la couleur de cheveux colorés, contenant, par rapport à son poids,
a. 0,01 à 4,00 % en poids d'au moins un alun, et
b. 0,01 à 5,00 % en poids d'au moins un sel organique ou inorganique de cuivre, de zinc, de fer (II), de calcium et/ou de magnésium,
- l'agent de traitement capillaire présentant un pH compris entre 4,0 et 5,0.

2. Agent de traitement capillaire selon la revendication 1, **caractérisé en ce que** le rapport en poids de l'au moins un alun a) à l'au moins un sel b) est compris entre 4:1 et 1:3, de préférence entre 3,5:1 et 1:2,5, de manière particulièrement préférée entre 3:1 et 1:2, et en particulier entre 2,5:1 et 1:1,5.

3. Agent de traitement capillaire selon l'une des revendications précédentes, **caractérisé en ce qu'**il présente un pH compris entre 4,1 et 4,9, de manière particulièrement préférée entre 4,2 et 4,8, et plus préférablement entre 4,3 et 4,7.

4. Agent de traitement capillaire selon l'une des revendications précédentes, **caractérisé en ce que** l'alun a) correspond à la formule M^{I}Al(SO₄)₂ dans laquelle M^{I} représente un ion potassium, sodium, ammonium ou guanidinium.

5. Agent de traitement capillaire selon l'une des revendications précédentes, **caractérisé en ce qu'**il contient, par rapport à son poids, 0,05 à 3,50 % en poids, de préférence 0,10 à 3,00 % en poids, de manière davantage préférée 0,15 à 2,50 % en poids, de manière particulièrement préférée 0,20 à 2,25 % en poids et de plus préférablement 0,25 à 2,00 % en poids d'au moins un alun, de préférence un alun de potassium, de sodium ou d'ammonium.

6. Agent de traitement capillaire selon l'une des revendications précédentes, **caractérisé en ce que** du lactate de calcium et/ou de magnésium est utilisé comme sel

7. Agent de traitement capillaire selon l'une des revendications précédentes, **caractérisé en ce qu'**il contient, par rapport à son poids, 0,05 à 20 % en poids d'au moins un tensioactif.

8. Agent de traitement capillaire selon la revendication 7, **caractérisé en ce qu'**il contient, par rapport à son poids, 1,00 à 20 % en poids d'au moins un tensioactif anionique.

9. Agent de traitement capillaire selon la revendication 7, **caractérisée en ce qu'**il contient, par rapport à son poids, 0,1 à 10 % en poids d'au moins un tensioactif cationique.

10. Agent de traitement capillaire selon l'une des revendications précédentes, **caractérisé en ce qu'**il contient, par rapport à son poids, 0,01 à 3 % en poids d'au moins une silicone.

11. Agent de traitement capillaire selon l'une des revendications précédentes, **caractérisé en ce qu'**il contient, par rapport à son poids, 0,01 à 3 % en poids d'au moins un polymère cationique.

12. Agent de traitement capillaire selon l'une des revendications précédentes, **caractérisé en ce qu'**il est appliqué avant ou après un traitement oxydant (coloration) de fibres kératiniques.

13. Procédé de protection de la couleur de fibres kératiniques colorées, dans lequel une composition cosmétique présentant un pH compris entre 4,0 et 5,0, et contenant, par rapport à son poids,
a. 0,01 à 4,00 % en poids d'au moins un alun, et
b. 0,01 à 5,00 % en poids d'au moins un sel organique ou inorganique de cuivre, de zinc, de fer (II), de calcium et/ou de magnésium, est appliquée sur les fibres kératiniques pendant une période de 5 secondes à 24 heures avant ou après la coloration.
